# FASCICULE DE BREVET EUROPEEN

(11) **EP 1 100 935 B1**
(45) Date de publication et mention de la délivrance du brevet: **09.09.2009**
(21) Numéro de dépôt: 99934813.9
(22) Date de dépôt: 29.07.1999
(51) Int. Cl.: C12N 15/70, C12N 1/21

(54) **SOUCHES MUTANTES DE E. COLI, ET LEUR UTILISATION POUR LA PRODUCTION DE POLYPEPTIDES RECOMBINANTS**
MUTANTENSTÄMMEN VON ESCHERICHIA COLI UND DEREN VERWENDUNG ZUR HERSTELLUNG VON REKOMBINANT-POLYPEPTIDEN
MUTANT E.COLI STRAINS, AND THEIR USE FOR PRODUCING RECOMBINANT POLYPEPTIDES

(30) Priorité: 07.08.1998 FR 9810197
(43) Date de publication de la demande: 23.05.2001
(73) Titulaire: CENTRE NATIONAL DE LA RECHERCHE SCIENTIFIQUE, 75016 Paris Cédex (FR)
(72) Inventeur: DREYFUS, Marc, F-75013 Paris (FR); LOPEZ, Pascal, F-38380 Saint-Laurent-du-Pont (FR)
(74) Mandataire: Grosset-Fournier, Chantal Catherine
(86) Numéro de dépôt international: PCT/FR1999/001879
(87) Numéro de publication internationale: WO 2000/008183

(56) Documents cités:
- EP-A- 0 178 863
- IOST I. ET AL.: "The stability of Escherichia coli lacZ mRNA depends upon the simultaneity of its synthesis and translation" EMBO JOURNAL, vol. 14, no. 13, 1995, pages 3252-3261, XP002101971 cité dans la demande
- KIDO M ET AL.: "RNase E polypeptides lacking a carboxyl-terminal half suppress a mukB mutation in Escherichia coli" JOURNAL OF BACTERIOLOGY, vol. 178, no. 13, juillet 1996 (1996-07), pages 3917-3925, XP002101972 cité dans la demande
- DATABASE PASCAL NO.: 97-0507561 [Online] INIST, CNRS (CENTRE NATIONAL DE LA RECHERCHE SCIENTIFIQUE), VANDOEUVRE-LES-NANCY, FR Thesis, mai 1995 (1995-05) IOST I: "Couplages entres la synthses, la traduction et la degradation des ARN messagers chez Escherichia coli: example de l'ARN messager lacZ" XP002101973
- MCDOWELL K.J. ET AL.: "The N-terminal domain of the rne gene-product has Rnase-E activity and is non-overlapping with the Arginine-rich RNA-binding site" JOURNAL OF MOLECULAR BIOLOGY, vol. 255, no. 3, 26 janvier 1996 (1996-01-26), pages 349-355, XP002121516 OXFORD GB
- LOPEZ P.J. ET AL.: "The C-terminal half of the Rnase E, which organizes the Escherichia coli degardosome, participates in mRNA degradation but not rRNA processing in vivo" MOLECULAR MICROBIOLOGY, vol. 33, no. 1, juillet 1999 (1999-07), pages 188-199, XP002121517 OXFORD GB

### LISTE DE SEQUENCES

(1) INFORMATION GENERALE:
   (i) DEPOSANT:
      (A) NOM: CNRS
      (B) RUE: 3, rue Michel-Ange
      (C) VILLE: PARIS
      (E) PAYS: FRANCE
      (F) CODE POSTAL: 75794 CEDEX 16
   (ii) TITRE DE L' INVENTION: Souches mutantes de *E. coli,* et leur utilisation pour la production de polypeptides recombinants
   (iii) NOMBRE DE SEQUENCES: 6
   (iv) FORME LISIBLE PAR ORDINATEUR:
      (A) TYPE DE SUPPORT: Floppy disk
      (B) ORDINATEUR: IBM PC compatible
      (C) SYSTEME D' EXPLOITATION: PC-DOS/MS-DOS
      (D) LOGICIEL: PatentIn Release #1.0, Version #1.25 (OEB)
(2) INFORMATION POUR LA SEQ ID NO: 1:
   (i) CARACTERISTIQUES DE LA SEQUENCE:
      (A) LONGUEUR: 3661 paires de bases
      (B) TYPE: acide nucléique
      (C) NOMBRE DE BRINS: double
      (D) CONFIGURATION: linéaire
   (ii) TYPE DE MOLECULE: ADN (génomique)
   (ix) CARACTERISTIQUE ADDITIONELLE:
      (A) NOM/CLE: CDS
      (B) EMPLACEMENT: 441..3623
   (xi) DESCRIPTION DE LA SEQUENCE: SEQ ID NO: 1:
   GAAAAAACTG TGAGTAAGCG GGTGATAAAT GGTAAAAGTC ATCTTGCTAT AACAAGGCTT      60
   GCAGTGGAAT AATGAGGCCG TTTCCGTGTC CATCCTTGTT AAAACAAGAA ATTTTACGGA     120
   ATAACCCATT TTGCCCGACC GATCATCCAC GCAGCAATGG CGTAAGACGT ATTGATCTTT     180
   CAGGCAGTTA GCGGGCTGCG GGTTGCAGTC CTTACCGGTA GATGGAAATA TTTCTGGAGA     240
   GTAATACCCA GTCTGTTTCT TGTATAATTG CGCTGTTTTT CCGCATGAAA AACGGGCAAC     300
   CGACACTCTG CGCCTCTTTG AGCTGACGAT AACCGTGAGG TTGGCGACGC GACTAGACAC     360
   GAGGCCATCG GTTCACACCC GGAAAGGCGT TACTTTGCCC GCAGCTTAGT CGTCAATGTA     420
   AGAATAATGA GTAAGTTACG ATG AAA AGA ATG TTA ATC AAC GCA ACT CAG         470
                         Met Lys Arg Met Leu Ile Asn Ala Thr Gln
                          1               5                   10
   CAG GAA GAG TTG CGC GTT GCC CTT GTA GAT GGG CAG CGT CTG TAT GAC     518
   Gln Glu Glu Leu Arg Val Ala Leu Val Asp Gly Gln Arg Leu Tyr Asp
                       15                  20                  25
   CTG GAT ATC GAA AGT CCA GGG CAC GAG CAG AAA AAG GCA AAC ATC TAC     566
   Leu Asp Ile Glu Ser Pro Gly His Glu Gln Lys Lys Ala Asn Ile Tyr
                   30                  35                  40
   AAA GGT AAA ATC ACC CGC ATT GAA CCG AGT CTG GAA GCT GCT TTT GTT     614
   Lys Gly Lys Ile Thr Arg Ile Glu Pro Ser Leu Glu Ala Ala Phe Val
               45                  50                  55
   GAT TAC GGC GCT GAA CGT CAC GGT TTC CTC CCA CTA AAA GAA ATT GCC     662
   Asp Tyr Gly Ala Glu Arg His Gly Phe Leu Pro Leu Lys Glu Ile Ala
           60                  65                  70
   CGC GAA TAT TTC CCT GCT AAC TAC AGT GCT CAT GGT CGT CCC AAC ATT     710
   Arg Glu Tyr Phe Pro Ala Asn Tyr Ser Ala His Gly Arg Pro Asn Ile
   75                  80                  85                  90
   AAA GAT GTG TTG CGT GAA GGT CAG GAA GTC ATT GTT CAG ATC GAT AAA     758
   Lys Asp Val Leu Arg Glu Gly Gln Glu Val Ile Val Gln Ile Asp Lys
                   95                  100                 105
   GAA GAG CGC GGC AAC AAA GGC GCG GCA TTA ACC ACC TTT ATC AGT CTG     806
   Glu Glu Arg Gly Asn Lys Gly Ala Ala Leu Thr Thr Phe Ile Ser Leu
               110                 115                 120
   GCG GGT AGC TAT CTG GTT CTG ATG CCG AAC AAC CCG CGC GCG GGT GGC     854
   Ala Gly Ser Tyr Leu Val Leu Met Pro Asn Asn Pro Arg Ala Gly Gly
           125                 130                 135
   ATT TCT CGC CGT ATC GAA GGC GAC GAC CGT ACC GAA TTA AAA GAA GCA     902
   Ile Ser Arg Arg Ile Glu Gly Asp Asp Arg Thr Glu Leu Lys Glu Ala
       140                 145                 150
   CTG GCA AGC CTT GAA CTG CCG GAA GGC ATG GGG CTT ATC GTG CGC ACC     950
   Leu Ala Ser Leu Glu Leu Pro Glu Gly Met Gly Leu Ile Val Arg Thr
   155                 160                 165                 170
   GCT GGC GTC GGC AAA TCT GCT GAG GCG CTG CAA TGG GAT TTA AGC TTC     998
   Ala Gly Val Gly Lys Ser Ala Glu Ala Leu Gln Trp Asp Leu Ser Phe
                   175                 180                 185
   CGT CTG AAA CAC TGG GAA GCC ATC AAA AAA GCC GCT GAA AGC CGC CCG     1046
   Arg Leu Lys His Trp Glu Ala Ile Lys Lys Ala Ala Glu Ser Arg Pro
               190                 195                 200
   GCC CCG TTC CTG ATT CAT CAG GAG AGC AAC GTA ATC GTT CGC GCA TTC     1094
   Ala Pro Phe Leu Ile His Gln Glu Ser Asn Val Ile Val Arg Ala Phe
           205                 210                 215
   CGC GAT TAC TTA CGT CAG GAC ATC GGC GAA ATC CTT ATC GAT AAC CCG     1142
   Arg Asp Tyr Leu Arg Gln Asp Ile Gly Glu Ile Leu Ile Asp Asn Pro
       220                 225                 230
   AAA GTG CTC GAA CTG GCA CGT CAG CAT ATC GCT GCA TTA GGT CGC CCG      1190
   Lys Val Leu Glu Leu Ala Arg Gln His Ile Ala Ala Leu Gly Arg Pro
   235                 240                 245                 250
   GAT TTC AGC AGC AAA ATC AAA CTG TAC ACC GGC GAG ATC CCG CTG TTC      1238
   Asp Phe Ser Ser Lys Ile Lys Leu Tyr Thr Gly Glu Ile Pro Leu Phe
                   255                 260                 265
   AGC CAC TAC CAG ATC GAG TCA CAG ATC GAG TCC GCC TTC CAG CGT GAA      1286
   Ser His Tyr Gln Ile Glu Ser Gln Ile Glu Ser Ala Phe Gln Arg Glu
               270                 275                 280
   GTT CGT CTG CCG TCT GGT GGT TCC ATT GTT ATC GAC AGC ACC GAA GCG      1334
   Val Arg Leu Pro Ser Gly Gly Ser Ile Val Ile Asp Ser Thr Glu Ala
           285                 290                 295
   TTA ACG GCC ATC GAC ATC AAC TCC GCA CGC GCG ACC CGC GGC GGC GAT      1382
   Leu Thr Ala Ile Asp Ile Asn Ser Ala Arg Ala Thr Arg Gly Gly Asp
       300                 305                 310
   ATC GAA GAA ACC GCG TTT AAC ACT AAC CTC GAA GCT GCC GAT GAG ATT      1430
   Ile Glu Glu Thr Ala Phe Asn Thr Asn Leu Glu Ala Ala Asp Glu Ile
   315                 320                 325                 330
   GCT CGT CAG CTG CGC CTG CGT GAC CTC GGC GGC CTG ATT GTT ATC GAC      1478
   Ala Arg Gln Leu Arg Leu Arg Asp Leu Gly Gly Leu Ile Val Ile Asp
                   335                 340                 345
   TTC ATC GAC ATG ACG CCA GTA CGC CAC CAG CGT GCG GTA GAA AAC CGT      1526
   Phe Ile Asp Met Thr Pro Val Arg His Gln Arg Ala Val Glu Asn Arg
               350                 355                 360
   CTG CGT GAA GCG GTG CGT CAG GAC CGT GCG CGT ATT CAA ATC AGC CAT      1574
   Leu Arg Glu Ala Val Arg Gln Asp Arg Ala Arg Ile Gln Ile Ser His
           365                 370                 375
   ATT TCT CGC TTT GGC CTG CTG GAA ATG TCC CGT CAG CGC CTG AGC CCA      1622
   Ile Ser Arg Phe Gly Leu Leu Glu Met Ser Arg Gln Arg Leu Ser Pro
       380                 385                 390
   TCA CTG GGT GAA TCC AGT CAT CAC GTT TGT CCG CGT TGT TCT GGT ACT      1670
   Ser Leu Gly Glu Ser Ser His His Val Cys Pro Arg Cys Ser Gly Thr
   395                 400                 405                 410
   GGC ACC GTG CGT GAC AAC GAA TCG CTG TCG CTC TCT ATT CTG CGT CTG      1718
   Gly Thr Val Arg Asp Asn Glu Ser Leu Ser Leu Ser Ile Leu Arg Leu
                   415                 420                 425
   ATC GAA GAA GAA GCG CTG AAA GAG AAC ACC CAG GAA GTT CAC GCC ATT      1766
   Ile Glu Glu Glu Ala Leu Lys Glu Asn Thr Gln Glu Val His Ala Ile
               430                 435                 440
   GTT CCT GTG CCA ATC GCT TCT TAC CTG CTG AAT GAA AAA CGT TCT GCG      1814
   Val Pro Val Pro Ile Ala Ser Tyr Leu Leu Asn Glu Lys Arg Ser Ala
           445                 450                 455
   GTA AAT GCC ATT GAA ACT CGT CAG GAC GGT GTG CGC TGT GTA ATT GTG     1862
   Val Asn Ala Ile Glu Thr Arg Gln Asp Gly Val Arg Cys Val Ile Val
       460                 465                 470
   CCA AAC GAT CAG ATG GAA ACC CCG CAC TAC CAC GTG CTG CGC GTG CGT     1910
   Pro Asn Asp Gln Met Glu Thr Pro His Tyr His Val Leu Arg Val Arg
   475                 480                 485                 490
   AAA GGG GAA GAA ACC CCA ACC TTA AGC TAC ATG CTG CCG AAG CTG CAT     1958
   Lys Gly Glu Glu Thr Pro Thr Leu Ser Tyr Met Leu Pro Lys Leu His
                   495                 500                 505
   GAA GAA GCG ATG GCG CTG CCG TCT GAA GAA GAG TTC GCT GAA CGT AAG     2006
   Glu Glu Ala Met Ala Leu Pro Ser Glu Glu Glu Phe Ala Glu Arg Lys
               510                 515                 520
   CGT CCG GAA CAA CCT GCG CTG GCA ACC TTT GCC ATG CCG GAT GTG CCG     2054
   Arg Pro Glu Gln Pro Ala Leu Ala Thr Phe Ala Met Pro Asp Val Pro
           525                 530                 535
   CCT GCG CCA ACG CCA GCT GAA CCT GCC GCG CCT GTT GTA GCT CCA GCA     2102
   Pro Ala Pro Thr Pro Ala Glu Pro Ala Ala Pro Val Val Ala Pro Ala
       540                 545                 550
   CCG AAA GCT GCA CCG GCA ACA CCA GCA GCT CCT GCA CAA CCT GGG CTG     2150
   Pro Lys Ala Ala Pro Ala Thr Pro Ala Ala Pro Ala Gln Pro Gly Leu
   555                 560                 565                 570
   TTG AGC CGC TTC TTC GGC GCA CTG AAA GCG CTG TTC AGC GGT GGT GAA     2198
   Leu Ser Arg Phe Phe Gly Ala Leu Lys Ala Leu Phe Ser Gly Gly Glu
                   575                 580                 585
   GAA ACC AAA CCG ACC GAG CAA CCA GCA CCG AAA GCA GAA GCG AAA CCG     2246
   Glu Thr Lys Pro Thr Glu Gln Pro Ala Pro Lys Ala Glu Ala Lys Pro
               590                 595                 600
   GAA CGT CAA CAG GAT CGT CGC AAG CCT CGT CAG AAC AAC CGC CGT GAC     2294
   Glu Arg Gln Gln Asp Arg Arg Lys Pro Arg Gln Asn Asn Arg Arg Asp
           605                 610                 615
   CGT AAT GAG CGC CGC GAC ACC CGT AGT GAA CGT ACT GAA GGC AGC GAT     2342
   Arg Asn Glu Arg Arg Asp Thr Arg Ser Glu Arg Thr Glu Gly Ser Asp
       620                 625                 630
   AAT CGC GAA GAA AAC CGT CGT AAT CGT CGC CAG GCA CAG CAG CAG ACT     2390
   Asn Arg Glu Glu Asn Arg Arg Asn Arg Arg Gln Ala Gln Gln Gln Thr
   635                 640                 645                 650
   GCC GAG ACG CGT GAG AGC CGT CAG CAG GCT GAG GTA ACG GAA AAA GCG     2438
   Ala Glu Thr Arg Glu Ser Arg Gln Gln Ala Glu Val Thr Glu Lys Ala
                   655                 660                 665
   CGT ACC GCC GAC GAG CAG CAA GCG CCG CGT CGT GAA CGT AGC CGC CGC     2486
   Arg Thr Ala Asp Glu Gln Gln Ala Pro Arg Arg Glu Arg Ser Arg Arg
               670                 675                 680
   CGT AAT GAT GAT AAA CGT CAG GCG CAA CAA GAA GCG AAG GCG CTG AAT 2534
   Arg Asn Asp Asp Lys Arg Gln Ala Gln Gln Glu Ala Lys Ala Leu Asn
   685 690 695
   GTT GAA GAG CAA TCT GTT CAG GAA ACC GAA CAG GAA GAA CGT GTA CGT 2582
   Val Glu Glu Gln Ser Val Gln Glu Thr Glu Gln Glu Glu Arg Val Arg
   700 705 710
   CCG GTT CAG CCG CGT CGT AAA CAG CGT CAG CTC AAT CAG AAA GTG CGT 2630
   Pro Val Gln Pro Arg Arg Lys Gln Arg Gln Leu Asn Gln Lys Val Arg
   715 720 725 730
   TAC GAG CAA AGC GTA GCC GAA GAA GCG GTA GTC GCA CCG GTG GTT GAA 2678
   Tyr Glu Gln Ser Val Ala Glu Glu Ala Val Val Ala Pro Val Val Glu
   735 740 745
   GAA ACT GTC GCT GCC GAA CCA ATT GTT CAG GAA GCG CCA GCT CCA CGC 2726
   Glu Thr Val Ala Ala Glu Pro Ile Val Gln Glu Ala Pro Ala Pro Arg
   750 755 760
   ACA GAA CTG GTG AAA GTC CCG CTG CCA GTC GTA GCG CAA ACT GCA CCA 2774
   Thr Glu Leu Val Lys Val Pro Leu Pro Val Val Ala Gln Thr Ala Pro
   765 770 775
   GAA CAG CAA GAA GAG AAC AAT GCT GAT AAC CGT GAC AAC GGT GGC ATG 2822
   Glu Gln Gln Glu Glu Asn Asn Ala Asp Asn Arg Asp Asn Gly Gly Met
   780 785 790
   CCG CGT CGT TCT CGC CGC TCG CCT CGT CAC CTG CGC GTA AGT GGT CAG 2870
   Pro Arg Arg Ser Arg Arg Ser Pro Arg His Leu Arg Val Ser Gly Gln
   795 800 805 810
   CGT CGT CGT CGC TAT CGT GAC GAG CGT TAT CCA ACC CAG TCG CCA ATG 2918
   Arg Arg Arg Arg Tyr Arg Asp Glu Arg Tyr Pro Thr Gln Ser Pro Met
   815 820 825
   CCG TTG ACC GTA GCG TGC GCG TCT CCG GAA CTG GCC TCT GGC AAA GTC 2966
   Pro Leu Thr Val Ala Cys Ala Ser Pro Glu Leu Ala Ser Gly Lys Val
   830 835 840
   TGG ATC CGC TAT CCA ATT GTA CGT CCG CAA GAT GTA CAG GTT GAA GAG 3014
   Trp Ile Arg Tyr Pro Ile Val Arg Pro Gln Asp Val Gln Val Glu Glu
   845 850 855
   CAG CGC GAA CAG GAA GAA GTA CAT GTG CAG CCG ATG GTG ACT GAG GTC 3062
   Gln Arg Glu Gln Glu Glu Val His Val Gln Pro Met Val Thr Glu Val
   860 865 870
   CCT GTC GCC GCC GCT ATC GAA CCG GTT GTT AGC GCG CCA GTT GTT GAA 3110
   Pro Val Ala Ala Ala Ile Glu Pro Val Val Ser Ala Pro Val Val Glu
   875 880 885 890
   GAA GTG GCC GGT GTC GTA GAA GCC CCC GTT CAG GTT GCC GAA CCG CAA 3158
   Glu Val Ala Gly Val Val Glu Ala Pro Val Gln Val Ala Glu Pro Gln
   895 900 905
   CCG GAA GTG GTT GAA ACG ACG CAT CCT GAA GTG ATC GCT GCC GCG GTA      3206
   Pro Glu Val Val Glu Thr Thr His Pro Glu Val Ile Ala Ala Ala Val
               910                 915                 920
   ACT GAA CAG CCG CAG GTG ATT ACC GAG TCT GAT GTT GCC GTA GCC CAG      3254
   Thr Glu Gln Pro Gln Val Ile Thr Glu Ser Asp Val Ala Val Ala Gln
           925                 930                 935
   GAA GTT GCA GAA CAA GCA GAA CCG GTG GTT GAA CCG CAG GAA GAG ACG      3302
   Glu Val Ala Glu Gln Ala Glu Pro Val Val Glu Pro Gln Glu Glu Thr
       940                 945                 950
   GCA GAT ATT GAA GAA GTT GTC GAA ACT GCT GAG GTT GTA GTT GCT GAA      3350
   Ala Asp Ile Glu Glu Val Val Glu Thr Ala Glu Val Val Val Ala Glu
   955                 960                 965                 970
   CCT GAA GTT GTT GCT CAA CCT GCC GCG CCA GTA GTC GCT GAA GTC GCA      3398
   Pro Glu Val Val Ala Gln Pro Ala Ala Pro Val Val Ala Glu Val Ala
                   975                 980                 985
   GCA GAA GTT GAA ACG GTA GCT GCG GTC GAA CCT GAG GTC ACC GTT GAG      3446
   Ala Glu Val Glu Thr Val Ala Ala Val Glu Pro Glu Val Thr Val Glu
               990                 995                 1000
   CAT AAC CAC GCT ACC GCG CCA ATG ACG CGC GCT CCA GCA CCG GAA TAT      3494
   His Asn His Ala Thr Ala Pro Met Thr Arg Ala Pro Ala Pro Glu Tyr
           1005                1010                1015
   GTT CCG GAG GCA CCG CGT CAC AGT GAC TGG CAG CGC CCT ACT TTT GCC      3542
   Val Pro Glu Ala Pro Arg His Ser Asp Trp Gln Arg Pro Thr Phe Ala
       1020                1025                1030
   TTC GAA GGT AAA GGT GCC GCA GGT GGT CAT ACG GCA ACA CAT CAT GCC      3590
   Phe Glu Gly Lys Gly Ala Ala Gly Gly His Thr Ala Thr His His Ala
   1035                1040                1045                1050
   TCT GCC GCT CCT GCG CGT CCG CAA CCT GTT GAG TAATAATTAG CTCAAAGTAA    3643
   Ser Ala Ala Pro Ala Arg Pro Gln Pro Val Glu
                   1055                1060
   TCAAGCCCTG GTAACTGC                                                  3661
(2) INFORMATION POUR LA SEQ ID NO: 2:
   (i) CARACTERISTIQUES DE LA SEQUENCE:
      (A) LONGUEUR: 1061 acides aminés
      (B) TYPE: acide aminé
      (D) CONFIGURATION: linéaire
   (ii) TYPE DE MOLECULE: protéine
   (xi) DESCRIPTION DE LA SEQUENCE: SEQ ID NO: 2:
   Met Lys Arg Met Leu Ile Asn Ala Thr Gln Gln Glu Glu Leu Arg Val
   1 5 10 15
   Ala Leu Val Asp Gly Gln Arg Leu Tyr Asp Leu Asp Ile Glu Ser Pro
   20 25 30
   Gly His Glu Gln Lys Lys Ala Asn Ile Tyr Lys Gly Lys Ile Thr Arg
   35 40 45
   Ile Glu Pro Ser Leu Glu Ala Ala Phe Val Asp Tyr Gly Ala Glu Arg
   50 55 60
   His Gly Phe Leu Pro Leu Lys Glu Ile Ala Arg Glu Tyr Phe Pro Ala
   65 70 75 80
   Asn Tyr Ser Ala His Gly Arg Pro Asn Ile Lys Asp Val Leu Arg Glu
   85 90 95
   Gly Gln Glu Val Ile Val Gln Ile Asp Lys Glu Glu Arg Gly Asn Lys
   100 105 110
   Gly Ala Ala Leu Thr Thr Phe Ile Ser Leu Ala Gly Ser Tyr Leu Val
   115 120 125
   Leu Met Pro Asn Asn Pro Arg Ala Gly Gly Ile Ser Arg Arg Ile Glu
   130 135 140
   Gly Asp Asp Arg Thr Glu Leu Lys Glu Ala Leu Ala Ser Leu Glu Leu
   145 150 155 160
   Pro Glu Gly Met Gly Leu Ile Val Arg Thr Ala Gly Val Gly Lys Ser
   165 170 175
   Ala Glu Ala Leu Gln Trp Asp Leu Ser Phe Arg Leu Lys His Trp Glu
   180 185 190
   Ala Ile Lys Lys Ala Ala Glu Ser Arg Pro Ala Pro Phe Leu Ile His
   195 200 205
   Gln Glu Ser Asn Val Ile Val Arg Ala Phe Arg Asp Tyr Leu Arg Gln
   210 215 220
   Asp Ile Gly Glu Ile Leu Ile Asp Asn Pro Lys Val Leu Glu Leu Ala
   225 230 235 240
   Arg Gln His Ile Ala Ala Leu Gly Arg Pro Asp Phe Ser Ser Lys Ile
   245 250 255
   Lys Leu Tyr Thr Gly Glu Ile Pro Leu Phe Ser His Tyr Gln Ile Glu
   260 265 270
   Ser Gln Ile Glu Ser Ala Phe Gln Arg Glu Val Arg Leu Pro Ser Gly
   275 280 285
   Gly Ser Ile Val Ile Asp Ser Thr Glu Ala Leu Thr Ala Ile Asp Ile
   290 295 300
   Asn Ser Ala Arg Ala Thr Arg Gly Gly Asp Ile Glu Glu Thr Ala Phe
   305 310 315 320
   Asn Thr Asn Leu Glu Ala Ala Asp Glu Ile Ala Arg Gln Leu Arg Leu
   325 330 335
   Arg Asp Leu Gly Gly Leu Ile Val Ile Asp Phe Ile Asp Met Thr Pro
   340 345 350
   Val Arg His Gln Arg Ala Val Glu Asn Arg Leu Arg Glu Ala Val Arg
   355 360 365
   Gln Asp Arg Ala Arg Ile Gln Ile Ser His Ile Ser Arg Phe Gly Leu
   370 375 380
   Leu Glu Met Ser Arg Gln Arg Leu Ser Pro Ser Leu Gly Glu Ser Ser
   385 390 395 400
   His His Val Cys Pro Arg Cys Ser Gly Thr Gly Thr Val Arg Asp Asn
   405 410 415
   Glu Ser Leu Ser Leu Ser Ile Leu Arg Leu Ile Glu Glu Glu Ala Leu
   420 425 430
   Lys Glu Asn Thr Gln Glu Val His Ala Ile Val Pro Val Pro Ile Ala
   435 440 445
   Ser Tyr Leu Leu Asn Glu Lys Arg Ser Ala Val Asn Ala Ile Glu Thr
   450 455 460
   Arg Gln Asp Gly Val Arg Cys Val Ile Val Pro Asn Asp Gln Met Glu
   465 470 475 480
   Thr Pro His Tyr His Val Leu Arg Val Arg Lys Gly Glu Glu Thr Pro
   485 490 495
   Thr Leu Ser Tyr Met Leu Pro Lys Leu His Glu Glu Ala Met Ala Leu
   500 505 510
   Pro Ser Glu Glu Glu Phe Ala Glu Arg Lys Arg Pro Glu Gln Pro Ala
   515 520 525
   Leu Ala Thr Phe Ala Met Pro Asp Val Pro Pro Ala Pro Thr Pro Ala
   530 535 540
   Glu Pro Ala Ala Pro Val Val Ala Pro Ala Pro Lys Ala Ala Pro Ala
   545 550 555 560
   Thr Pro Ala Ala Pro Ala Gln Pro Gly Leu Leu Ser Arg Phe Phe Gly
   565 570 575
   Ala Leu Lys Ala Leu Phe Ser Gly Gly Glu Glu Thr Lys Pro Thr Glu
   580 585 590
   Gln Pro Ala Pro Lys Ala Glu Ala Lys Pro Glu Arg Gln Gln Asp Arg
   595 600 605
   Arg Lys Pro Arg Gln Asn Asn Arg Arg Asp Arg Asn Glu Arg Arg Asp
   610 615 620
   Thr Arg Ser Glu Arg Thr Glu Gly Ser Asp Asn Arg Glu Glu Asn Arg
   625 630 635 640
   Arg Asn Arg Arg Gln Ala Gln Gln Gln Thr Ala Glu Thr Arg Glu Ser
   645 650 655
   Arg Gln Gln Ala Glu Val Thr Glu Lys Ala Arg Thr Ala Asp Glu Gln
   660 665 670
   Gln Ala Pro Arg Arg Glu Arg Ser Arg Arg Arg Asn Asp Asp Lys Arg
   675 680 685
   Gln Ala Gln Gln Glu Ala Lys Ala Leu Asn Val Glu Glu Gln Ser Val
   690 695 700
   Gln Glu Thr Glu Gln Glu Glu Arg Val Arg Pro Val Gln Pro Arg Arg
   705 710 715 720
   Lys Gln Arg Gln Leu Asn Gln Lys Val Arg Tyr Glu Gln Ser Val Ala
   725 730 735
   Glu Glu Ala Val Val Ala Pro Val Val Glu Glu Thr Val Ala Ala Glu
   740 745 750
   Pro Ile Val Gln Glu Ala Pro Ala Pro Arg Thr Glu Leu Val Lys Val
   755 760 765
   Pro Leu Pro Val Val Ala Gln Thr Ala Pro Glu Gln Gln Glu Glu Asn
   770 775 780
   Asn Ala Asp Asn Arg Asp Asn Gly Gly Met Pro Arg Arg Ser Arg Arg
   785 790 795 800
   Ser Pro Arg His Leu Arg Val Ser Gly Gln Arg Arg Arg Arg Tyr Arg
   805 810 815
   Asp Glu Arg Tyr Pro Thr Gln Ser Pro Met Pro Leu Thr Val Ala Cys
   820 825 830
   Ala Ser Pro Glu Leu Ala Ser Gly Lys Val Trp Ile Arg Tyr Pro Ile
   835 840 845
   Val Arg Pro Gln Asp Val Gln Val Glu Glu Gln Arg Glu Gln Glu Glu
   850 855 860
   Val His Val Gln Pro Met Val Thr Glu Val Pro Val Ala Ala Ala Ile
   865 870 875 880
   Glu Pro Val Val Ser Ala Pro Val Val Glu Glu Val Ala Gly Val Val
   885 890 895
   Glu Ala Pro Val Gln Val Ala Glu Pro Gln Pro Glu Val Val Glu Thr
   900 905 910
   Thr His Pro Glu Val Ile Ala Ala Ala Val Thr Glu Gln Pro Gln Val
   915 920 925
   Ile Thr Glu Ser Asp Val Ala Val Ala Gln Glu Val Ala Glu Gln Ala
       930                 935                 940
   Glu Pro Val Val Glu Pro Gln Glu Glu Thr Ala Asp Ile Glu Glu Val
   945                 950                 955                 960
   Val Glu Thr Ala Glu Val Val Val Ala Glu Pro Glu Val Val Ala Gln
                   965                 970                 975
   Pro Ala Ala Pro Val Val Ala Glu Val Ala Ala Glu Val Glu Thr Val
               980                 985                 990
   Ala Ala Val Glu Pro Glu Val Thr Val Glu His Asn His Ala Thr Ala
           995                 1000                1005
   Pro Met Thr Arg Ala Pro Ala Pro Glu Tyr Val Pro Glu Ala Pro Arg
       1010                1015                1020
   His Ser Asp Trp Gln Arg Pro Thr Phe Ala Phe Glu Gly Lys Gly Ala
   1025                1030                1035                1040
   Ala Gly Gly His Thr Ala Thr His His Ala Ser Ala Ala Pro Ala Arg
                   1045                1050                1055
   Pro Gln Pro Val Glu
               1060
(2) INFORMATION POUR LA SEQ ID NO: 3:
   (i) CARACTERISTIQUES DE LA SEQUENCE:
      (A) LONGUEUR: 27 paires de bases
      (B) TYPE: acide nucléique
      (C) NOMBRE DE BRINS: simple
      (D) CONFIGURATION: linéaire
   (ii) TYPE DE MOLECULE: ADN
   (xi) DESCRIPTION DE LA SEQUENCE: SEQ ID NO: 3:
      GGGCTGCAGT TTCCGTGTCC ATCCTTG 27
(2) INFORMATION POUR LA SEQ ID NO: 4:
   (i) CARACTERISTIQUES DE LA SEQUENCE:
      (A) LONGUEUR: 26 paires de bases
      (B) TYPE: acide nucléique
      (C) NOMBRE DE BRINS: simple
      (D) CONFIGURATION: linéaire
   (ii) TYPE DE MOLECULE: ADN
   (xi) DESCRIPTION DE LA SEQUENCE: SEQ ID NO: 4:
      GGGAGATCTT GATTACTTTG AGCTAA 26
(2) INFORMATION POUR LA SEQ ID NO: 5:
   (i) CARACTERISTIQUES DE LA SEQUENCE:
      (A) LONGUEUR: 42 paires de bases
      (B) TYPE: acide nucléique
      (C) NOMBRE DE BRINS: double
      (D) CONFIGURATION: linéaire
   (ii) TYPE DE MOLECULE: ADN
   (xi) DESCRIPTION DE LA SEQUENCE: SEQ ID NO: 5:
      CTGCAGATAG CCCGCCTAAT GAGCGGGCTT TTTTTTCTGC AG 42
(2) INFORMATION POUR LA SEQ ID NO: 6:
   (i) CARACTERISTIQUES DE LA SEQUENCE:
      (A) LONGUEUR: 21 paires de bases
      (B) TYPE: acide nucléique
      (C) NOMBRE DE BRINS: simple
      (D) CONFIGURATION: linéaire
   (ii) TYPE DE MOLECULE: ADN
   (xi) DESCRIPTION DE LA SEQUENCE: SEQ ID NO: 6:
      GCGGTGGTTA AGAAACCAAA C 21

## Description

La présente invention a pour objet certaines souches mutantes de *E*. *coli,* et leur utilisation pour la mise en oeuvre de procédés de production de polypeptides recombinants.

L'étude des gènes des organismes supérieurs, des micro-organismes, et des virus, requiert presque invariablement, outre le clonage de ces gènes, la production massive de leurs produits (protéines), afin par exemple d'obtenir des anticorps ou de réaliser des études biochimiques ou cristallographiques.

D'un point de vue appliqué, l'utilisation en médecine de nombreux peptides et protéines humaines nécessite aussi l'expression des gènes correspondants dans des organismes hétérologues.

Bien que des systèmes d'expression aient été mis au point dans divers hôtes eucaryotes (notamment chez les levures, les insectes, et les cellules de primates), l'hôte le plus utilisé pour ces stratégies d'expression reste la bactérie *Escherichia coli* (*E. coli*)*.* La liste des protéines présentant un intérêt biotechnologique ou pharmacologique qui sont produites chez *E*. *coli* est longue; citons les exemples classiques de l'insuline humaine et de l'hormone de croissance humaine.

Le système d'expression le plus connu chez les procaryotes a été développé aux USA par les groupes de Studier et de Richardson dans les années 80 (Tabor et Richardson, 1985; Studier et Moffat, 1986). Il est basé sur l'exploitation des propriétés de l'ARN polymérase de T7 (à savoir de l'ARN polymérase codée par le bactériophage T7). Cette enzyme, qui peut être exprimée dans les cellules de *E*. *coli* sans toxicité, reconnaît un promoteur très spécifique. Tout gène intéressant (dit gène cible) peut être transcrit de façon très efficace, dès lors qu'il est placé en aval de ce promoteur et introduit dans une cellule de *E*. *coli* exprimant la polymérase de T7.

Cependant, en termes d' expression, les résultats demeurent aléatoires. Si certains gènes cibles peuvent être convenablement surexprimés, d'autres ne le sont que médiocrement, voire pas du tout.

Il ressort du travail antérieur des Inventeurs qu'une des principales causes de ces échecs réside dans l'instabilité spécifique des ARNm synthétisés par l'ARN polymérase de T7, qui entraîne une diminution du nombre de polypeptides synthétisés par message (Lopez *et al.,* 1994 ; Iost et Dreyfus, 1994, 1995). Cette instabilité est la conséquence de la grande vitesse d'élongation de l'ARN polymérase de T7 (Makarova *et al.,* 1995). En effet, la vitesse d'élongation de la polymérase de T7, au contraire de l'ARN polymérase bactérienne, est beaucoup plus élevée que la vitesse de traduction des ARNm par les ribosomes. L'ARNm naissant est donc nu sur la plus grande partie de sa longueur, et est de ce fait facilement attaqué par les nucléases, à savoir principalement chez *E*. *coli* la ribonucléase dite E (ou RNase E), dont la séquence en aminoacides est décrite par Casaregola *et al.* (Casaregola *et al.,* 1992, 1994).

La RNase E est une enzyme essentielle de *E*. *coli ;* elle est impliquée à la fois dans la dégradation des ARNm et dans la maturation des ARN ribosomaux (ARNr). Les mutations dans le domaine catalytique (à savoir dans la partie N-terminale de la RNase E) affectent ces deux fonctions à la fois, et ralentissent, voire arrêtent la croissance de *E*. *coli* (Cohen et McDowall, 1997).

En revanche, les délétions dans la partie C-terminale de la RNase E n'affectent pas la viabilité de *E*. *coli*. En effet, en recherchant des révertants de mutations dans une protéine (MukB) nécessaire à la ségrégation des chromosomes après réplication, Kido *et al.* ont obtenu diverses mutations viables dans le gène *rne*, codant la RNase E chez *E*. *coli,* qui provoquent la synthèse d'une RNase E tronquée dans sa partie C-terminale (Kido *et al.,* 1996). Ces auteurs ont conclu de ces expériences que la partie C-terminale de la RNase E n'est pas essentielle pour la viabilité de *E*. *coli*. Ils ont de plus émis l'hypothèse que la suppression des mutations *mukB* par troncature de la RNase E, reflète le fait que la RNase E tronquée est moins efficace que l'enzyme sauvage pour dégrader l'ARNm *mukB*. Stabilisé, celui-ci permettrait alors une plus forte synthèse de la protéine MukB mutante, corrigeant ainsi le phénotype associé à la mutation. Toutefois, cette stabilisation du messager *mukB* n'a pas été démontrée, et d'autres auteurs ont proposé une interprétation entièrement différente pour expliquer l'effet suppresseur de la troncature de la RNase E sur les mutations *mukB* (Cohen et McDowal, 1997). Ces auteurs postulent en effet une interaction directe entre la RNase E et MukB. A l'origine de cette idée se trouve le fait que la RNase E présente une analogie très significative avec la myosine eucaryote (Casaregola *et al. ,* 1992; McDowall *et al. ,* 1993), ce qui suggère qu'outre son activité RNase, elle pourrait, comme MukB, jouer un rôle structural.

La présente invention découle de la démonstration par les Inventeurs du fait que la troncature de la RNase E provoque une stabilisation en masse de l'ARNm cellulaire - considéré dans sa globalité - , ainsi que de la majorité des ARNm individuels qu'ils ont examinés, sans pour autant gêner significativement la maturation des ARNr (Lopez *et al.,* 1999).

En cela, l'effet de la délétion est très différent de celui d'une mutation dans la région N-terminale, comme la mutation *ams* (Ono et Kuwano, 1979), rebaptisée *rne*1 (Babitzke et Kushner, 1991) qui confère à la RNase E une activité thermosensible. Par exemple, à 37°C, cette dernière mutation provoque une augmentation modérée de la durée de vie des ARNm (x1,5 fois en moyenne; la durée de vie des ARNm est ici définie comme le temps pendant lequel ils peuvent servir de matrice à la synthèse protéique (Mudd *et al.,* 1990a)) mais elle cause aussi un important retard à la maturation des ARNr (estimée d'après la méthode de "Northern" ; voir Lopez *et al. ,* 1994) et elle ralentit la croissance par un facteur 2. Au contraire, la délétion de la partie C-terminale de la RNase E, notamment des acides aminés 586 à 1061 de cette dernière, provoque une stabilisation plus importante des ARNm (2 fois en moyenne), sans entraîner de retard à la maturation des ARNr et sans ralentir la croissance. A posteriori donc, il est vraisemblable que le défaut de croissance que l'on observe avec les mutations N-terminales de la RNase E, est uniquement due à l'incapacité des cellules à maturer l'ARNr.

En résumé, les délétions de la partie C-terminale de la RNase E n'ont pas d'effet sur l'activité du domaine catalytique, à en juger par la maturation rapide de l'ARNr. Cette maturation rapide explique que les cellules contenant une telle délétion soient viables. En revanche, la délétion stabilise globalement les ARNm, peut être parce qu'elle empêche l'association de la RNase E avec d'autres enzymes au sein d'une structure multiprotéique, le "dégradosome", qui pourrait être nécessaire à la dégradation des ARNm (Carpousis *et al. ,* 1994 ; Miczack *et al*, 1996 ; Py *et al*., 1996 ; Kido *et al.,* 1996 ; Cohen et McDowall, 1997). Le point important, du point de vue de l'invention, est que grâce à ces délétions il est possible d' obtenir des souches de *E*. *coli* présentant une stabilité accrue des ARNm, tout en conservant une croissance normale.

Les Inventeurs ont également pu mettre en évidence que la stabilisation des ARNm due à la délétion de la partie C-terminale de la RNase E, n'est pas uniforme, mais qu'elle est plus marquée pour les ARNm les moins stables. Ainsi qu'on l'a vu, tel est souvent le cas des ARNm des gènes "cibles" dans les systèmes d'expression. La contribution de ces ARNm à la synthèse protéique totale est donc augmentée par la présence de la délétion. Ainsi des souches de *E*. *coli* comportant une telle délétion expriment des polypeptides exogènes recombinants avec des rendements nettement accrus (notamment entre environ 3 à 25 fois) par rapport aux rendements d'expression de ces polypeptides recombinants par des souches de *E. coli* ne comportant pas cette mutation, et ce en particulier dans le cas où l'expression desdits polypeptides recombinants est placée sous le contrôle de l'ARN polymérase de T7.

La présente invention a donc pour but de fournir de nouveaux procédésde production par *E*. *coli* de polypeptides ou protéines recombinants, notamment d'intérêt biologique ou pharmaceutique, ayant des rendements de production nettement supérieurs à ceux des procédés décrits jusqu'à présent.

La présente invention a également pour but de fournir de nouvelles souches de *E*. *coli* pour la mise en oeuvre des procédés susmentionnés, ainsi que les méthodes de préparation de telles souches.

La présente invention a pour objet l'utilisation de souches de *Escherichia coli* (*E. coli*) pour la mise en oeuvre d'un procédé de production de polypeptides (ou protéines) recombinants exogènes déterminés, le gène codant la RNase E desdites souches comportant une mutation telle que l'enzyme produite lors de l'expression de ce gène muté ne possède plus l'activité de dégradation des BARN messagers (ARNm), mais conserve l'activité de maturation de l'ARN ribosomal (ARNr) de la RNase E, cette mutation n'affectant pas la croissance desdites souches de *E*. *coli,* **caractérisée en ce que** la mutation consiste en la substitution ou la délétion d'un ou plusieurs nucléotides de la région du gène codant pour la partie C-terminale de la RNase E délimitée par le nucléotide situé à la position 1935 et le nucléotide situé en position 3623 de la séquence d'ADN codant la RNase E représentée par SEQ ID NO 1, de manière à provoquer la modification ou la délétion d'au moins un aminoacide jusqu'à la totalité des 563 derniers aminoacides de la partie C-terminale de la RNase E représentée par SEQ ID NO 2.

L'invention a plus particulièrement pour objet l'utilisation susmentionnée de souches de *E. coli* telles que définies ci-dessus, **caractérisée en ce que** la mutation correspond à la substitution de la guanine G en position 2196 de SEQ NID NO 1, par une thymidine T, de manière à créer un codon stop TAA situé aux positions 2196 à 2198 de SEQ ID NO 1.

Avantageusement, les souches susmentionnées de *E*. *coli* mutées, utilisées dans le cadre de la présente invention, contiennent un système d'expression inductible exogène, sous le contrôle duquel est placée l'expression des polypeptides recombinants déterminés, notamment le système d'expression inductible utilisant l'ARN polymérase du bactériophage T7.

L'invention concerne également les souches de *E. coli* transformées de manière à ce qu'elles contiennent un système d'expression inductible exogène, et dont le gène codant la RNase E comporte une mutation telle que l'enzyme produite lors de l'expression de ce gène muté ne possède plus l'activité de dégradation des ARN, mais conserve l'activité de maturation de l'ARN ribosomal (ARN) de la RNase E, cette mutation n'affectant pas significativement la croissance desdites souches de *E. coli,* et consistant en la substitution ou la délétion d'un ou plusieurs nucléotides de la région du gène codant pour la partie C-terminale de la RNase E délimitée par le nucléotide situé à la position 1935 et le nucléotide situé en position 3623 de la séquence d'ADN codant la RNase E représentée par SEQ ID NO 1, de manière à provoquer la modification ou la délétion d'au moins un aminoacide jusqu'à la totalité des 563 derniers aminoacides de la partie C-terminale de la RNase E représentée par SEQ ID NO 2.

L'invention a plus particulièrement pour objet les souches de *E*. *coli* telles que décrites ci-dessus, **caractérisées en ce que** le système d'expression inductible utilise l'ARN polymérase codée par le bactériophage T7.

L'invention a plus particulièrement pour objet les souches de *E. coli* telles que définies ci-dessus, **caractérisées en ce que** la mutation correspond à la substitution de la guanine G en position 2196 de SEQ ID NO 1, par une thymidine T, de manière à créer un codon stop TAA situé aux positions 2196 à 2198 de SEQ ID NO 1.

L'invention a également pour objet les souches de *E*. *coli* telles que décrites ci-dessus, **caractérisées en ce que** le système d'expression inductible contrôle la transcription d'une séquence d'ADN codant un ou plusieurs polypeptides recombinants déterminés.

L'invention concerne également tout procédéde production de polypeptides recombinants déterminés, **caractérisé en ce qu**'il comprend :
- une étape de transformation de souches de *E. coli* dont le gène codant la RNase E comporte une mutation telle que l'enzyme produite lors de l'expression de ce gène muté ne possède plus l'activité de dégradation des ARNm, mais conserve l'activité de maturation de l'ARN ribosomal (ARNr) de la RNase E, cette mutation n'affectant pas significativement la croissance desdites souches de *E. coli*, et consistant en la substitution ou la délétion d'un ou plusieurs nucléotides de la région du gène codant pour la partie C-terminale de la RNase E délimitée par le nucléotide situé à la position 1935 et le nucléotide situé en position 3623 de la séquence d'ADN codant la RNase E représentée par SEQ ID NO 1, de manière à provoquer la modification ou la délétion d'au moins un aminoacide jusqu'à la totalité des 563 derniers aminoacides de la partie C-terminale de la RNase E représentée par SEQ ID NO 2, avec un vecteur, notamment un plasmide, contenant la séquence nucléotidique codant un ou plusieurs polypeptides recombinants,
- la mise en culture des souches de *E*. *coli* transformées obtenues lors de l'étape précédente,pendant un temps suffisant pour permettre l'expression du ou des polypeptides recombinants dans les cellules de *E. coli*,
- et la récupération du ou des polypeptides recombinants produits lors de l'étape précédente, le cas échéant après purification de ces derniers, notamment par chromatographie, électrophorèse ou précipitation sélective.

L'invention a plus particulièrement pour objet tout procédéde production de polypeptides recombinants déterminés, tel que défini ci-dessus, **caractérisé en ce qu**'il comprend :
- une étape de transformation de souches de *E*. *coli* telles que décrites ci-dessus, avec un vecteur, notamment un plasmide, contenant la séquence nucléotidique codant un ou plusieurs polypeptides recombinants, de manière à obtenir des souches de *E*. *coli* susmentionnées, dans lesquelles la transcription de ladite séquence nucléotidique codant un ou plusieurs polypeptides recombinants est placée sous le contrôle d'un système d'expression inductible,
- la mise en culture des souches de *E*. *coli* transformées obtenues lors de l'étape précédente, et induction dudit système d'expression, pendant un temps suffisant pour permettre l'expression du ou des polypeptides recombinants dans les cellules de *E*. *coli,* l'induction dudit système d'expression étant notamment effectué en provoquant la synthèse de l'ARN polymérase de T7 lorsque ledit système d'expression fait appel à cette polymérase; cette synthèse peut notamment être provoquée en ajoutant de l'IPTG au milieu de culture, ou en élevant la température, suivant que le gène codant pour cette ARN polymérase est placé sous le contrôle d'un promoteur régulé par le répresseur *lac* (Studier et Moffat, 1986), ou sous le contrôle d'un promoteur thermoinductible (Tabor et Richardson, 1985),
- et la récupération du ou des polypeptides recombinants produits lors de l'étape précédente.

Un procédé général pour l'obtention de souches mutantes de *E*. *coli* telles que décrites ci-dessus, et susceptibles de pouvoir être utilisées dans le cadre de la présente invention, comprend les étapes suivantes :
- préparation d'un plasmide contenant un gène *me* comportant une mutation telle que décrite ci-dessus, et dans lequel le promoteur dudit gène *rne* est supprimé,
- introduction du plasmide obtenu à l'étape précédente dans une souche de *E. coli* comportant un système d'expression inductible, ainsi qu'une mutation chromosomique dans le gène *rne* conférant une propriété particulière à ladite souche de *E*. *coli,* telle que la mutation dite *rne1* (Ono and Kuwano, 1979) rendant la croissance de l'hôte thermosensible, et permettant de sélectionner l'acquisition de la mutation souhaitée du gène *rne* sur le chromosome de *E*. *coli*,
- mise en culture des souches de *E*. *coli* ainsi transformées, et sélection des souches de *E*. *coli* possédant la propriété particulière susmentionnée, à savoir des clones résultant de la recombinaison homologue qui permet de remplacer ladite mutation chromosomique par la séquence homologue correspondant au gène *rne* muté dudit plasmide, notamment sélection des clones thermorésistants dans le cas où la mutation chromosomique est ladite mutation *rne1*,
- élimination du plasmide des clones sélectionnés, et identification parmi ces clones de ceux comportant le gène *rne* muté susmentionné, notamment en analysant par électrophorèse la longueur du polypeptide RNase E tronqué, codé par le gène *rne* muté susmentionné, produit par les cellules mutantes de *E*. *coli.*

L'invention sera davantage illustrée à l'aide de la description détaillée qui suit de la préparation d'une souche mutée de *E. coli* selon l'invention, et de son utilisation pour la production de polypeptides déterminés.

### 1) Construction d'un gène rne muté contenant un codon STOP au 586ème codon

Cette position particulière a été choisie parce que la troncature ainsi créée dans la RNase E est formellement équivalente à celle qui résulte de la mutation spontanée *smbB131* (rebaptisée ici *rne131*) obtenue par Kido *et al* (1996). Celle-ci est une délétion de 2 nucléotides au 586ème codon, entraînant un changement de phase de lecture suivie d'un arrêt après une traduction supplémentaire de 32 codons sans rapport avec la séquence normale de la RNase E.

Pour construire un tel gène, le nucléotides G2196 de la séquence est substitué par T, créant un codon TAA (stop) aux nucléotides 2196-2198 de SEQ ID NO1 (cette mutation sera conventionnellement notée ici "G2196T").

Pour créer la substitution souhaitée, le gène *rne* sauvage est d'abord sous-cloné dans le 'phagemide' pEMBL8⁺ (Dente *et al.,* 1983). Pour cela, on amplifie à partir du génome de *E. coli* la totalité de la séquence transcrite du gène *rne* à l'aide des amorces suivantes :
SEQ ID NO 3 : 5' GGG*CTGCAG***TTTCCGTGTCCATCCTTG** 3' (la séquence en gras correspond aux nucléotides (nt) 81-98 de SEQ ID NO 1; la séquence en italique est la séquence de reconnaissance de l'enzyme *Pst*I), et
SEQ ID NO 4 : 5' GGG*AGATC****T*TGATTACTTTGAGCTAA** 3' (la séquence en gras est complémentaire aux nt 3630 à 3647 de SEQ ID NO 1; la séquence en italique est reconnue par l'enzyme *Bgl*II).

Le fragment amplifié est alors digéré par les enzymes *Bgl*II et *Pst*I (ces enzymes n'ont pas de sites de coupure à l'intérieur de la séquence *rne*), puis inséré entre les sites *Bam*HI et *Pst*I de pEMBL8⁺ (rappelons que les sites *Bam*HI et *Bgl*II peuvent être ligaturés entre eux). On note que la séquence *rne* ainsi clonée est dépourvue de son promoteur.

On élimine toute transcription parasite provenant du vecteur en introduisant ensuite dans le site *Pst*I de la séquence obtenue, et dans le même sens que le gène *rne*, le fragment synthétique suivant: :
SEQ ID NO 5 : *CTGCAG***ATAGCCCGCCTAATGAGCGGGCTTTTTTTT***CTGCAG*
(la séquence en gras correspond à un terminateur transcriptionnel très efficace, celui de l'opéron tryptophane (Christie *et al.,* 1981), et les extrémités en italique correspondent à la séquence reconnue par *Pst*I). Ces précautions garantissent que la séquence *rne* portée par le plasmide ne peut être transcrite à partir de promoteurs plasmidiques, et donc que la RNase E ne peut être synthétisée à partir du plasmide. L'intérêt de ce point apparaîtra plus loin. Dans la suite, le plasmide ainsi obtenu est nommé pRNE.

La substitution désirée (G- > T) est alors introduite dans le plasmide pRNE en utilisant la méthode classique de mutagenèse dirigée décrite par Kunkel (Kunkel *et al*., 1987). Pour cela, le plasmide pRNE est introduit dans la souche RZ1032 (Hfr KL16 PO/45 (*lysA61-62 dut1 ung1 thi1 relA1 supE44 zbd-279::*Tn*10*). Les mutations *dut1* et *ung1* présentes dans cette souche provoquent l'incorporation de desoxyuridine (dU) au lieu de thymidine (T) dans l'ADN. Les cellules sont alors surinfectées par le phage M13 'helper' K07 (Pharmacia), ce qui provoque l'accumulation dans le milieu de 'phages' comportant la séquence de pRNE sous forme de simple brin, avec dU à la place de T. Après déprotéinisation, cette matrice simple brin est hybridée avec l'oligonucléotide synthétique suivant :
SEQ ID NO 6 : GCGGTGGTTAAGAAACCAAAC
correspondant aux positions 2188 à 2208 de SEQ ID NO 1 (le 'T' qu'on souhaite incorporer à la place de G est indiqué en gras), puis l'hybride est converti en ADN double brin par incubation avec la polymérase de Klenow, la ligase de T4, de l'ATP et les quatre dNTP (Kunkel *et al*., 1987). L'hybride double brin est alors introduit dans XL1, une souche de *E*. *coli* classiquement utilisée pour le clonage (Stratagene). Cette souche est sauvage pour les gènes *dut* et *ung*, et par conséquent le brin initial comportant dU à la place de T, sera dégradé. La grande majorité des colonies de XL1 obtenues comportent donc la mutation désirée dans le plasmide pRNE.

Choisissant quatre candidats, on s'assure alors que la mutation souhaitée est bien présente et que le plasmide n'en comporte pas d'autre. Pour cela, on détermine à l'aide d'amorces oligonucléotidiques appropriées, la séquence de la région *Afl*II-*Nru*I (nt 1931-2345 de SEQ ID NO 1), et on sélectionne les candidats qui ne comportent dans cette région que la seule mutation souhaitée, à l'exclusion de toute autre modification. Le fragment 4*fl*II-*Nru*I provenant d'un tel candidat est alors isolé et on le substitue au fragment *Afl*II-*Nru*I du plasmide pRNE initial (non mutagénisé). On obtient ainsi un plasmide comportant la mutation souhaitée; ce plasmide est appelé par la suite pRNE-STOP.

### 2) Introduction de la mutation créant un stop au codon 586 de la RNase E, sur le chromosome de BL21(DE3), une souche exprimant l'ARN polymérase du bactériophage T7.

*Principe général*. La mutation souhaitée (G2196T) ne produit pas de changement de phénotype par rapport au gène sauvage. Pour l'introduire sur le chromosome, on est donc conduit à procéder en deux étapes: on introduit d'abord dans la RNase E une mutation faux-sens au codon 66 (mutation dite *ams,* ou *rne1*; Ono & Kuwano, 1979). Cette mutation correspond à la transition G636A, suivant la numérotation de SEQ ID NO 1 (McDowall *et al.,* 1993). Elle diminue la stabilité thermique de la RNase E, empêchant la croissance à haute température.

Ensuite, on introduit dans la souche obtenue le plasmide pRNE-stop, et on sélectionne les cellules qui peuvent à nouveau croître à haute température. Rappelons que le gène *me-stop* porté par le plasmide, n'étant pas transcrit, ne peut conduire à la synthèse d'une RNase E fonctionnelle. Toutefois, grâce à une double recombinaison homologue, le plasmide peut apporter au gène *rne* chromosomique la séquence sauvage à la position 636 (mutation A636G), rétablissant du même coup la croissance à haute température. La région d'homologie entre le plasmide et la région *rne* chromosomique s'étendant sur près de 1500 nt en aval de la mutation G2196T portée par le plasmide, cette dernière mutation a une forte probabilité d'être transférée sur le chromosome en même temps que A636G. Le plasmide est ensuite éliminé; le résultat est une souche comportant la seule mutation G2196T dans le gène chromosomique de la RNase E.

*Préparation d'un dérivé rne1 (ams) de la souche BL21(DE3)*. BL21(DE3) est l'hôte habituel des systèmes d'expression bactériens fondés sur la transcription de gènes hétérologues par la polymérase de T7 (Studier & Moffat, 1986). Les outils permettant d'introduire la mutation *rne1* dans n'importe quel contexte génétique ont été décrits, en particulier pour BL21(DE3) et ses dérivés (Mudd *et al.,* 1990b; Iost & Dreyfus, 1995). En bref, on part d'une souche bactérienne (CH1828), comportant la mutation *ams*/*rne1* ainsi qu'un gène de résistance à la tétracycline inséré dans un locus chromosomique (*zce-726*) situé à peu de distance du gène *rne*. Utilisant la technique classique dite de transduction par le bactériophage P1 (Silhavy *et al*., 1984), une région du chromosome de CH1828 longue de quelques dizaines de milliers de nucléotides et entourant le locus *zce-726*, est transférée dans BL21(DE3), en sélectionnant l'acquisition de la résistance à la tétracycline (Tet^{R}). Une forte proportion (environ 50%) de ces clones Tet^{R} montrent également une croissance thermosensible, ce qui indique qu'ils ont reçu également l'allèle *rne1*. La souche résultante est appelée BL21(DE3)*rne1*.

*Introduction de la mutation G2196T sur le chromosome de BL21(DE3)* On transforme BL21(DE3)*rne1* avec le plasmide pRNE-stop (et, à titre de contrôle, avec le plasmide pEMBL8⁺ de départ), puis après croissance en milieu liquide complet (milieu LB; (Miller, 1972)) à 30°C, on étale environ 10⁵ bactéries sur des boîtes de Pétri contenant le même milieu gélosé, puis on incube à 42°C. Pour les bactéries contrôle, on n'observe aucune croissance après 24h (la mutation *ams*/*rne1* ne reverse pas spontanément). Au contraire, les bactéries transformées avec pRNE-stop montrent un grand nombre de clones thermorésistants, provenant de la réversion de la mutation *rne1* chromosomique grâce à la séquence sauvage apportée par le plasmide. On choisit alors une douzaine de ces clones thermorésistants, et on élimine le plasmide de ces candidats en les cultivant sans ampicilline pendant une vingtaine de générations en milieu LB liquide (42°C). L'ampicilline est nécessaire au maintien des plasmides dérivés de pEMBL8⁺; en son absence, le plasmide ségrège très facilement (Dreyfus, 1988). Après réisolement sur milieu LB gélosé des candidats ainsi traités, on vérifie la perte du plasmide en testant que les colonies individuelles ne peuvent plus croître en présence d'ampicilline.

Reste à identifier ceux des révertants thermorésistants - la majorité - qui, en même temps que la séquence sauvage à la position 636, ont aussi acquis la mutation G2196T. On procède en deux étapes. D'abord, les candidats sont réisolés sur milieu minimum gélosé (nous utilisons le milieu M63B1 avec du glycérol pour source de carbone; (Miller, 1972)); à titre de témoins, la souche initiale BL21(DE3) et le mutant thermosensible BL21(DE3)*rne1* sont également étalés sur les mêmes boîtes. Celles-ci sont incubées à 43°C. La mutation G2196T provoque un faible retard de croissance dans ces conditions extrêmes; les recombinants recherchés conduisent donc à des colonies plus petites que les cellules BL21(DE3) sauvages, ce qui permet un premier crible pour la recherche de ces recombinants. Le test final fait appel à la détermination directe de la taille du polypeptide RNase E, grâce à la technique immunologique dite de 'Western' (Sambrook *et al.,* 1989). En bref, on fait croître les différents candidats (ainsi que les deux témoins mentionnés ci-dessus) en milieu LB liquide. Quand la densité optique des cultures à 600 nm atteint 0.5, on récolte les cellules. Celles-ci sont resuspendues dans un tampon phosphate et lysées par sonication. Après élimination des débris, on dose les protéines dans l'extrait cellulaire (Bradford, 1976), puis on soumet 20 µg du mélange protéique à une électrophorèse suivant la technique de Laemmli (Laemmli, 1970), en utilisant un gel de polyacrylamide à 7,5%. Cette technique permet la séparation des protéines suivant leur taille. Après électrophorèse, le mélange protéique est électrotransféré sur une membrane de nitrocellulose. On sature ensuite la membrane avec des protéines non spécifiques, puis on l'incube avec une dilution (1/10.000) d'un anticorps polyclonal contre la RNase E élevé chez le lapin. On détecte les régions de la membrane ayant fixé l'anticorps anti-RNase E, en incubant celle-ci avec un anticorps de chèvre élevé contre les IgG de lapin, et couplé à l'enzyme peroxydase. La présence de la peroxydase sur la membrane est elle même révélée grâce à la technique d'électrochimioluminescence (ECL), en utilisant un kit commercialisé par Amersham. Cette technique permet de déterminer à quelle position a migré le polypeptide RNase E synthétisé par chacun des candidats, et donc la taille de ce polypeptide. En particulier, la réduction de taille occasionnée par la mutation G2196T est immédiatement visible dans ces tests. Plus de la moitié des candidats thermorésistants obtenus dans cette expérience possèdent la mutation désirée.

### Cas particulier de la mutation rne131.

Le protocole décrit ci-dessus a été aussi utilisé pour introduire sur le chromosome de BL21(DE3) les mutations spontanées (telle *rne131*) isolées par Kido *et al*., et conduisant également à la synthèse d'une RNase E tronquée. Toutefois, ces mutations étant dès le départ localisées sur le chromosome, le protocole s'en trouve simplifié.

La souche BZ31 (Kido *et al. ,* 1996) porte la mutation *rne131*. Grâce à la transduction par le bactériophage P1 (Silhavy *et al*., 1984; voir plus haut) on transpose dans BL21(DE3)*rne1* la région du chromosome de BZ31 entourant le locus *rne* en sélectionnant les transductants capables de croître à 42°C. On vérifie ensuite que ces clones synthétisent bien un polypeptide RNase E tronqué en utilisant la technique de "Western" décrite ci-dessus. Tous les candidats essayés (6/6, ou 100%) ont acquis la modification désirée. Incidemment, on peut vérifier que, comme on s'y attend au vu de l'expérience de construction de BL21(DE3)*rne1*, 50% des transductants thermorésistants ont aussi acquis le locus *zce-726* sauvage et sont donc redevenus sensibles à la tétracycline (Tet^{S}). On choisit par la suite un candidat Tet^{S}, appelé BL21(DE3)*rne131*.

### 3) Utilisation des souches BL21(DE3)rneG2196T ou BL21(DE3)rne131 pour l'expression efficace de gènes placés sous le contrôle du promoteur T7.

*Principe*. Les mutations *rneG2196T* ou *rne131* provoquent la stabilisation globale des ARNm par un facteur 2 environ. Toutefois, cette stabilisation n'est pas uniforme pour tous les ARNm. En particulier, sans doute à cause des propriétés particulières de l'ARN polymérase de T7 (cette enzyme a une vitesse d'élongation bien plus élevée que celle des ribosomes qui traduisent le message; voir ci-dessus), les ARNm synthétisés par cette enzyme semblent d'avantage stabilisés que la plupart des ARNm cellulaires. Il en résulte que la proportion des protéines totales constituée par les produits de ces ARNm particuliers, s'accroît lorsque qu'une mutation telle que *rneG2196T* ou *rne131* est présente dans la cellule. Cette observation est à la base de la présente invention. Nous donnons ci-dessous quelques exemples.

*Evaluation quantitative de l'invention: le gène lacZ comme système modèle*. Voici quelques années, les Inventeurs ont décrit la construction d'un dérivé de BL21(DE3), appelé ENS134, qui comporte une copie du gène *lacZ* insérée dans la région *malA* du chromosome (Iost & Dreyfus, 1995; Lopez et *al*., 1994). Ce gène, qui code pour un enzyme de *E*. *coli -* la β-galactosidase - dont l'expression est particulièrement facile à quantifier (Miller, 1972), est placé sous le contrôle du promoteur T7. Il est suivi par un gène codant pour un ARNt particulier, l'ARNt^{Arg5} de *E. coli,* dont l'expression donne une mesure commode du niveau de transcription (Lopez *et al*., 1994). Ce système bien défini permet des mesures particulièrement reproductibles sur la stabilité d'un ARNm particulier synthétisé par la polymérase de T7, ainsi que sur le rendement polypeptidique correspondant. A titre de test, la mutation *rne131* a été introduite dans ENS134 comme décrit ci dessus pour BL21(DE3). Nous avons fait croître les cellules de ENS134 ou de son dérivé portant la mutation à 37°C En ce qui concerne le milieu de culture, nous avons utilisé un milieu synthétique riche ou un milieu minimum (Neidhart *et al. ,* 1974), en présence d' IPTG (isopropyl β-D-thiogalactopyranoside ; il s'agit d'un inducteur dont la présence est nécessaire à la synthèse de polymérase de T7 dans BL21(DE3); Studier & Moffat, 1986). On récolte les cellules en phase exponentielle, on les lyse par sonication, et on dose la β-galactosidase dans l'extrait cellulaire, soit en mesurant l'activité enzymatique, soit en examinant l'abondance du polypeptide β-galactosidase par électrophorèse suivant Laemmli. On observe que la présence de la mutation augmente l'expression de la β-galactosidase par un facteur 25 environ en milieu riche, ou 80 en milieu minimum acétate, sans que le niveau de transcription du gène en soit affecté. Ce résultat, obtenu avec un système modèle, illustre les possibilités de l'invention.

*Expression de gènes eucaryotes clonés chez E. coli*. Dans les systèmes d'expression basés sur les propriétés de la polymérase de T7, le gène à exprimer - généralement un gène eucaryote - est fusionné en aval du promoteur T7 et d'un site de fixation de ribosome (RBS) permettant la traduction chez *E*. *coli*. L'ensemble est inséré dans un plasmide multicopie dérivé de pBR322, appelé pET, et placé dans la souche BL21(DE3) ou dans l'un de ses dérivés (Dubendorff & Studier, 1991; Studier & Moffat, 1986; Studier *et al.,* 1990). Comme ci-dessus, l'expression du gène à exprimer - le gène 'cible' - est déclenchée par ajout de l'inducteur IPTG au milieu de culture, qui provoque la synthèse de polymérase de T7. Toutefois, au contraire du système modèle décrit ci-dessus, l'induction par l'IPTG ne peut être ici que transitoire, parce que la transcription du gène à partir du promoteur T7 est si active qu'elle tue les cellules lorsque ce promoteur est présent sur un plasmide multicopie.

On a introduit dans BL21(DE3), et dans BL21(DE3)*rne131* ou BL21(DE3)*rneG2196T* des plasmides pET comprenant un certain nombre de gènes eucaryotes, à savoir le gène *Krox-20* impliqué dans le développement précoce de la souris (Vesque & Charnay, 1992), le gène *engrailed-2* impliqué dans la morphogenèse de l'embryon de poulet (Logan *et al*., 1992), et le gène codant pour la protéase de HTLV1, un rétrovirus humain (Malik *et al*., 1988). On a fait croître les cellules jusqu'à une DO₆₀₀ d'environ 0.5, puis on ajoute l'IPTG. Les cellules ont été récoltées quatre heures plus tard. On réalise alors un extrait cellulaire qu'on analyse par électrophorèse, comme décrit ci-dessus. Dans les trois cas, la détection du produit du gène 'cible' se fait par la technique de 'Western' (les anticorps polyclonaux et monoclonaux élevés contre *Krox-20* et *engrailed-2*, respectivement, ont été décrits: (Patel *et al.,* 1989; Vesque & Charnay, 1992)). On peut observer que le produit du gène 'cible' est trois à dix fois plus abondant lorsque l'hôte est BL21(DE3)*rne131* plutôt que BL21(DE3).

### Bibliographie

Babitzke, P. & Kushner, S.R. (1991). The ams (altered mRNA stability) protein and ribonuclease E are encoded by the same structural gene of Escherichia coli. Proc. Natl. Acad. Sci. USA 88, 1-5.
Bradford, M. M. (1976). A rapid and sensitive method for the quantification of microgram quantities of proteins utilizing the principle of protein-dye binding. Anal. Biochem. 72, 248-254.
Carpousis, A. J., Van Houwe, G., Ehretsmann, C. & Krisch, H. M. (1994). Copurification of E.coli RNAase E and PNPase : Evidence for a specific association between two enzymes important in RNA processing and degradation. Cell 76, 889-900.
Casaregola S., Jacq A., Laoudj D., McGurk G., Margarson S., Tempete M., Novis V. et Holland I.B. (1992). Cloning and analysis of the entire E. coli ams gene. ams is identical to hmp1 and encodes a 114 kDa protein that migrates as a 180 kDa protein. J. Mol. Biol. 228, 30-40. Erratum (1994) J. Mol. Biol. 238, 867.
Christie, G. E., Farnham, P. J. & Platt, T. (1981). Synthetic sites for transcription termination and a functional comparaison with tryptophan operon termination sites in vitro. Proc. Natl. Acad. Sci. USA 78, 4180-4184.
Cohen, S.N. & Mc Dowall, K. J. (1997). RNase E: still a wonderfully mysterious enzyme. Mol. Microbiol. 23, 1099-1106.
Dente, L., Cesareni, G. & Cortese (1983). pEMBL: a new family of single-stranded plasmids. Nucl. Acids Res. 11, 1645-1655.
Dreyfus, M. (1988). What constitues the signal for the initiation of protein synthesis on Escherichia coli mRNAs? J. Mol. Biol. 204, 79-94.
Dubendorff, J. W. & Studier, W. F. (1991). Controlling basal expression in an inductible T7 expression system by blocking the target T7 promotor with lac repressor. J. Mol. Biol. 219, 45-59.
Iost, I. & Dreyfus, M. (1994). mRNAs can be stabilized by DEAD-box proteins. Nature. 372, 193-196.
Iost, I. & Dreyfus, M. (1995). The stability of the E. coli lacZ mRNA depends upon the simultaneity of its synthesis and translation. EMBO J. 14, 3252-3261.
Kido M., Yamanaka K., Mitani T., Niki H., Ogura T. and Hiraga S. (1996). RNase E polypeptides lacking a carboxyl-terminal half suppress a mukB mutation in Escherichia coli. J. Bacteriol., 178, 3917-3925.
Kunkel, T. A., Roberts, J. D. & Zakour, R. A. (1987). Rapid and efficient site-specific mutagenesis without phenotypic selection. In Methods in Enzymology, pp. 367-382.
Laemmli, U. K. (1970). Cleavage of structural proteins during assembly of the head of bacteriophage T4. Nature 227, 680-685.
Logan, C., Hanks, M., Noble-Topham, S., Nallainathan, D., Provart, N.J. & Joyner, A.L. (1992). Cloning and sequence comparison of the mouse, human and chicken engrailed genes reveal potential functional domains and regulatory regions. Dev. Genet. 13, 345-358.
Lopez, P. J., Iost, I. & Dreyfus, M. (1994). The use of a tRNA as an transcriptional reporter: the T7 late promoter is extremely efficient in Escherichia coli but its transcripts are poorly expressed. Nucl. Acids Res. 22, 1186-1193. Erratum 22, 2434.
Lopez, P. J., Marchand I., Joyce S.A. et Dreyfus M. (1999). The C-terminal half of RNase E, which organizes the Escherichia coli degradosome, participates in in mRNA degradation but not in RNA processing in vivo, Mol. Microbiol 33, 188-1999.
McDowall, K. J., Hernandez, R. G., Lin-Chao, S. and S. N:, Cohen. (1993). The ams-1 and rne-3071 temperature-sensitive mutations in the ams gene are close proximity to each other and cause substitutions within a domain that resembles a product of the Eschericia coli mre locus. J. Bacteriol. 175, 4245-4249.
Makarova, O.V., Makarov, E. M., Sousa, R. & Dreyfus, M. (1995). Transcribing Escherichia coli genes with mutant T7 RNA polymerases : stability of lacZ mRNA inversely correlates with polymerase speed. Proc. Natl. Acad. Sci. USA 92, 12250-12254.
Malik, K.T., Even, J., et Karpus A. (1988). Molecular cloning and complete nucleotide sequence of an adult T cell leukaemia virus type I (ATLV/HTLV-1) isolate of Carribean origin: relationship to other members of the ATLV/HTLV-1 subgroup. J. Gen. Virol. 69, 1695-1710.
Miczak, A., Kaberdin, V.R., Wei, C.L. & Lin-Chao, S. (1996). Proteins associated with RNase E in a multicomponent ribonucleolytic complex. Proc. Natl. Acad. Sci. USA 93, 3865-3869.
Miller, J. H. (1972). Eperiments in Molecular Biology, Cold Spring Harbor Laboratory Press, Cold Spring Harbor, New York.
Mudd, E. A., Carpousis, A.J. & Krisch, H. M. (1990a). E. coli RNase E has a role in the decay of bacteriophase T4 mRNA. Genes and Development 4, 873-881.
Mudd, E. A., Krisch, H. M. & Higgins, C. F. (1990b). RNase E, an endoribonuclease, has a general role in the chemical decay of Escherichia coli mRNA: evidence that rne and ams are the same genetic locus. Mol. Microbiol. 4, 2127-2135.
Neidhart, F. C., Bloch, P. L. & Smith, D. F. (1974). Culture medium for Enterobacteria. J. Bacteriol. 119, 736-747.
Ono, M. & Kuwano, M. (1979). A conditional lethal mutation in an E. coli strain with a longer chemical lifetime of messenger RNA. J. Mol. Biol. 129, 343-357.
Patel, N. H., Martin, B. E., Coleman, K. G., Poole, S. J., Ellis, M. C., Kornberg, T. B. & Goodman, C. S. (1989). Expression of engrailed proteins in arthropods, annelids, and chordates. Cell 58, 955-968.
Py, B., Higgins, C.F., Krish, H. M. & Carpousis, A.J. (1996). A DEAD-box RNA helicase in the Escherichia coli RNA degradosome. Nature 381, 169-172.
Sambrook, J., Fritsch, E. F. & Maniatis, T. (1989). Molecular cloning: a laboratory manual. (Sambrook, J., Fritsch, E. F. & Maniatis, T., Eds.), Cold Spring Harbor Press, Cold Spring Harbor.
Silhavy, T. J., Berman, M. L. & Enquist, L. W. (1984). Experiments with gene fusions, Cold Spring Harbor Laboratory, Cold Spring Harbor.
Studier, F. W. & Moffat, B. A. (1986). Use of bacteriophage T7 RNA polymerase to direct selective high-level expression of cloned genes. J. Mol. Biol. 189, 113-130.
Studier, F. W., Rosenberg, A. H., Dunn, J. J. & Dubendorff, J. W. (1990). Use of T7 RNA polymerase to direct expression of cloned genes. In Methods in Enzymology (Academic Press, ed.), Vol. 185, pp. 60-89.
Tabor, S. & Richardson, C.C. (1985). A bacteriophage T7 RNA polymerase/promoter system for controlled exclusive expression of specific genes. Proc. Natl. Acad. Sc. USA 82, 1074-1078.
Vesque, C. & Charnay, P. (1992). Mapping functional regions of the segment-specific transcription factor Krox-20. Nucl. Acids. Res. 20, 2485-2492.

## Revendications

1. Utilisation de souches de *Escherichia coli* (*E. coli*) pour la mise en oeuvre d'un procédé de production de polypeptides (ou protéines) recombinants exogènes déterminés, le gène codant la RNase E desdites souches comportant une mutation telle que l'enzyme produite lors de l'expression de ce gène muté ne possède plus l'activité de dégradation des BARN messagers (ARNm), mais conserve l'activité de maturation de l'ARN ribosomal (ARNr) de la RNase E, cette mutation n'affectant pas la croissance desdites souches de *E. coli,* **caractérisée en ce que** la mutation consiste en la substitution ou la délétion d'un ou plusieurs nucléotides de la région du gène codant pour la partie C-terminale de la RNase E délimitée par le nucléotide situé à la position 1935 et le nucléotide situé en position 3623 de la séquence d'ADN codant la RNase E représentée par SEQ ID NO 1, de manière à provoquer la modification ou la délétion d'au moins un aminoacide jusqu'à la totalité des 563 derniers aminoacides de la partie C-terminale de la RNase E représentée par SEQ ID NO 2.

2. Utilisation de souches de *E*. *coli* selon la revendication 1, **caractérisée en ce que** la mutation correspond à la substitution de la guanine G en position 2196 de SEQ ID NO 1, par une thymidine T, de manière à créer un codon stop TAA situé aux positions 2196 à 2198 de SEQ ID NO 1.

3. Utilisation de souches de *E. coli* selon l'une des revendications 1 ou 2, **caractérisée en ce que** lesdites souches contiennent un système d'expression inductible exogène, sous le contrôle duquel est placée l'expression des polypeptides recombinants déterminés.

4. Utilisation de souches de *E. coli* selon l'une des revendications 1 à 3, **caractérisée en ce que** lesdites souches contiennent le système d'expression utilisant l'ARN polymérase du bactériophage T7.

5. Souches de *E*. *coli* transformées de manière à ce qu'elles contiennent un système d'expression inductible exogène, et dont le gène codant la RNase E comporte une mutation telle que l'enzyme produite lors de l'expression de ce gène muté ne possède plus l'activité de dégradation des ARN, mais conserve l'activité de maturation de l'ARN ribosomal (ARNr) de la RNase E, cette mutation n'affectant pas la croissance desdites souches de *E. coli*, et consistant en la substitution ou la délétion d'un ou plusieurs nucléotides de la région du gène codant pour la partie C-terminale de la RNase E délimitée par le nucléotide situé à la position 1935 et le nucléotide situé en position 3623 de la séquence d'ADN codant la RNase E représentée par SEQ ID NO 1, de manière à provoquer la modification ou la délétion d'au moins un aminoacide jusqu'à la totalité des 563 derniers aminoacides de la partie C-terminale de la RNase E représentée par SEQ ID NO 2.

6. Souches de *E*. *coli* selon la revendication 5, **caractérisées en ce que** le système d'expression inductible utilise l'ARN polymérase du bactériophage T7.

7. Souches de *E*. *coli* selon l'une des revendications 5 ou 6, **caractérisées en ce que** la mutation correspond à la substitution de la guanine G en position 2196 de SEQ ID NO 1, par une thymidine T, de manière à créer un codon stop TAA situé aux positions 2196 à 2198 de SEQ ID NO 1.

8. Souches de *E*. *coli* selon l'une des revendications 5 à 7, **caractérisées en ce que** le système d'expression inductible contrôle la transcription d'une séquence d'ADN codant un ou plusieurs polypeptides recombinants déterminés.

9. Procédé de production de polypeptides recombinants déterminés, **caractérisé en ce qu'**il comprend :
- une étape de transformation de souches de *E*. *coli* dont le gène codant la RNase E comporte une mutation telle que l'enzyme produite lors de l'expression de ce gène muté ne possède plus l'activité de dégradation des ARNm, mais conserve l'activité de maturation de l'ARN ribosomal (ARNr) de la RNase E, cette mutation n'affectant pas la croissance desdites souches de *E*. *coli,* et consistant en la substitution ou la délétion d'un ou plusieurs nucléotides de la région du gène codant pour la partie C-terminale de la RNase E délimitée par le nucléotide situé à la position 1935 et le nucléotide situé en position 3623 de la séquence d'ADN codant la RNase E représentée par SEQ ID NO 1, de manière à provoquer la modification ou la délétion d'au moins un aminoacide jusqu'à la totalité des 563 derniers aminoacides de la partie C-terminale de la RNase E représentée par SEQ ID NO 2, avec un vecteur, notamment un plasmide, contenant la séquence nucléotidique codant un ou plusieurs polypeptides recombinants,
- la mise en culture des souches de *E. coli* transformées obtenues lors de l'étape précédente, pendant un temps suffisant pour permettre l'expression du ou des polypeptides recombinants dans les cellules de *E*. *coli,*
- et la récupération du ou des polypeptides recombinants produits lors de l'étape précédente, le cas échéant après purification de ces derniers, notamment par chromatographie, électrophorèse, ou précipitation sélective.

10. Procédé de production de polypeptides recombinants déterminés selon la revendication 9, **caractérisé en ce qu'**il comprend :
- une étape de transformation de souches de *E. coli* selon l'une des revendications 5 à 7, avec un vecteur, notamment un plasmide, contenant la séquence nucléotidique codant un ou plusieurs polypeptides recombinants, de manière à obtenir des souches de *E*. *coli* selon la revendication 8, dans lesquelles la transcription de ladite séquence nucléotidique codant un ou plusieurs polypeptides recombinants est placée sous le contrôle d'un système d'expression inductible,
- la mise en culture des souches de *E*. *coli* transformées obtenues lors de l'étape précédente, et induction dudit système d'expression, pendant un temps suffisant pour permettre l'expression du ou des polypeptides recombinants dans les cellules de *E*. *coli,*
- et la récupération du ou des polypeptides recombinants produits lors de l'étape précédente.

## Claims

1. Use of *Escherichia coli* (*E. coli*) strains for practicing a process for producing predetermined exogenous recombinant polypeptides (or proteins), whose gene coding RNase E comprises a mutation such that the enzyme produced upon expression of this mutated gene possesses no longer activity for degrading messenger RNA (m-RNA), but preserves the activity for maturation of ribosomal RNA (r-RNA) of the RNase E, said mutation not affecting growth of the said *E. coli* strains, **characterized in that** mutation consists of the substitution or deletion of one or several nucleotides from the region of the gene coding for C-terminal portion of RNase E delimited by the nucleotide situated at position 1935 and the nucleotide situated at position 3623 of the DNA sequence coding the RNase E represented by SEQ ID NO: 1, so as to cause the modification or the deletion of at least one, up to all, of the last 563 amino acids of the sequence of RNase E represented by SEQ ID NO: 2.

2. Use of *E. coli* strains according to claims 1, **characterized in that** the mutation corresponds to the substitution of the guanine G at position 2196 of SEQ ID NO:1, by a thymidine T, so as to create a stop codon TAA situated at the positions 2196 to 2198 of SEQ ID NO: 1.

3. Use of *E-coli* strains according to one of claims 1 or 2, **characterized in that** the said strains contain an exogenous inducible expression system, under the control of which is placed the expression of the predetermined recombinant polypeptides.

4. Use of *E-coli* strains according to one of claims 1 to 3, **characterized in that** the said strains contain an expression system using RNA polymerase of the T7 bacteriophage.

5. *E. coli* strains transformed such that they contain an exogenous inducible expression system, and whose gene coding RNase E comprises a mutation such that the enzyme produced upon expression of this mutated gene possesses no longer activity for degradation of m-RNA, but preserves the activity for maturation of ribosomal RNA (r-RNA) of the RNase E, said mutation not affecting growth of the said *E. coli* strains, consisting of the substitution or deletion of one or several nucleotides from the region of the gene coding for C-terminal portion of RNase E delimited by the nucleotide situated at position 1935 and the nucleotide situated at position 3623 of the DNA sequence coding the RNase E represented by SEQ ID NO: 1, so as to cause the modification or the deletion of at least one, up to all, of the last 563 amino acids of the sequence of RNase E represented by SEQ ID NO: 2.

6. *E. coli* strains according to claim 5, **characterized in that** the inducible expression system uses RNA polymerase of the T7 bacteriophage.

7. *E. coli* strains according to one of claims 5 or 6, **characterized in that** the mutation corresponds to the substitution of the guanine G at position 2196 of SEQ ID NO:1, by a thymidine T, so as to create a stop codon TAA situated at positions 2196 to 2198 of SEQ ID NO:1.

8. *E. coli* strains according to one of claims 5 to 7, **characterized in that** the inducible expression system controls the transcription of a DNA sequence coding one or several predetermined recombinant polypeptides.

9. Process for producing predetermined recombinant polypeptides, **characterized in that** it comprises:
- a step of transforming *E. coli* strains whose gene coding RNase E comprises a mutation such that enzyme produced upon expression of this mutated gene no longer possesses degradation activity for m-RIVA, but preserves the activity for maturation of ribosomal RNA (r-RNA) of the RNase E, said mutation not affecting growth of the said *E. coli* strains, consisting of the substitution or deletion of one or several nucleotides from the region of the gene coding for C-terminal portion of RNase E delimited by the nucleotide situated at position 1935 and the nucleotide situated at position 3623 of the DNA sequence coding the RNase E represented by SEQ ID NO: 1, so as to cause the modification or the deletion of at least one, up to all, of the last 563 amino acids of the sequence of RNase E represented by SEQ ID NO: 2, with a vector, especially a plasmid, containing the nucleotide sequence coding one or several recombinant polypeptides,
- culturing the transformed *E. coli* strains obtained in the preceding step, for a sufficient time to permit expression of the recombinant polypeptide or polypeptides in the *E. coli* cells,
- and recovery of the recombinant polypeptide or polypeptides produced during the preceding step, optionally after purification of these latter, especially by chromatography, electrophoresis, or selective precipitation.

10. Process for producing predetermined recombinant polypeptides according to claim 9, **characterized in that** it comprises:
- a step of transforming *E. coli* strains according to one of claims 5 to 7, with a vector, especially a plasmid, containing the nucleotide sequence coding one or several recombinant polypeptides, so as to obtain *E. coli* strains according to claim 8, in which transcription of the said nucleotide sequence coding one or several recombinant polypeptides is placed under control of an inducible expression system,
- culturing the transformed *E. coli* strains obtained during the preceding step, and inducing the said expression system, for a sufficient time to permit expression of the recombinant polypeptide or polypeptides in the *E. coli* cells,
- and recovery of the recombinant polypeptide or polypeptides produced during the preceding step.

## Patentansprüche

1. Verwendung von *Escherichia coli* (*E. coli*)-Stammen für die Durchführung eines Verfahrens für die Herstellung von bestimmten exogenen rekombinanten Polypeptiden (oder Proteinen), worin das Gen, daß für die RNase E der genannten Stammen kodiert, mit einer solchen Mutation, daß das während der Expression dieses mutierten Gens hergestellte Enzym nicht mehr die Abbauwirkung auf die Messenger-RNA (mRNA) besitzt, sondern die Reifungswirkung auf die ribosomale RNA (rRNA) der RNase E behaltet, worin diese Mutation das Wachstum der genannten *E. coli*-Stammen nicht beeinflußt, **dadurch gekennzeichnet, daß** die Mutation aus der Substituierung oder der Deletion von einem oder mehreren Nukleotiden der Genregion besteht, die für den C-terminalen Teil der RNase E zwischen dem Nukleotid in der Position 1935 und dem Nukleotid in der Position 3623 der DNA-Sequenz codiert, worin diese Sequenz selbst für die mit SEQ ID NO 1 dargestellte RNase E codiert, und zwar um die Veränderung oder Deletion von zumindest einer Aminosäure bis zur Gesamtheit der 563 letzten Aminosäuren des C-terminalen Teils der mit SEQ ID NO 2 dargestellten RNase E zu verursachen.

2. Verwendung von *E. coli*-Stammen nach Anspruch 1, **dadurch gekennzeichnet, daß** die Mutation einer Substitution von Guanin G in der Position 2196 von SEQ ID NO 1 durch eine Thymidin T entspricht, und zwar um einen zwischen den Positionen 2196 und 2198 von SEQ ID NO 1 liegenden Stopcodon TAA zu schaffen.

3. Verwendung von *E. coli*-Stammen nach irgendwelcher Anspruch 1 oder 2, **dadurch gekennzeichnt, daß** die genannten Stammen ein exogenes induktierbares Expressionssystem beinhalten, daß die Expression der bestimmten rekombinanten Polypeptide kontrolliert.

4. Verwendung von *E*. *coli*-Stammen nach irgendwelcher Anspruch 1 - 3, **dadurch gekennzeichnet, daß** die genannten Stammen das die RNA-Polymerase des Bakteriophages T7 verwendende Expressionssystem beinhalten.

5. *E*. *coli*-Stammen, die so transformiert sind, daß sie ein exogenes induktierbares Expressionssystem beinhalten, dessen Gen, daß für die RNase E codiert, eine solche Mutation beinhaltet, daß das Enzym, daß während der Expression dieses mutierten Gens hergestellt wird, nicht meht die Abbauwirkung auf die mRNA besitzt, sondern die Reifungswirkung auf die ribosomale RNA (rRNA) der RNase E behaltet, worin diese Mutation das Wachstum der genannten *E. coli*-Stammen nicht beinflußt, **dadurch gekennzeichnet, daß** die Mutation aus der Substituierung oder der Deletion von einem oder mehreren Nukleotiden der Genregion besteht, die für den C-terminalen Teil der RNase E zwischen dem Nukleotid in der Position 1935 und dem Nukleotid in der Position 3623 der DNA-Sequenz codiert, worin diese Sequenz selbst für die mit SEQ ID NO 1 dargestellte RNase E codiert, und zwar um die Veränderung oder Deletion von zumindest einer Aminosäure bis zur Gesamtheit der 563 letzten Aminosäuren des C-terminalen Teils der mit SEQ ID NO 2 dargestellen RNase E zu verursachen.

6. *E. coli*-Stammen nach Anspruch 5, **dadurch gekennzeichnet, daß** das induzierbare Expressionssystem die RNA-Polymerase des Bakteriophages T7 verwendet.

7. *E. coli*-Stammen nach irgendwelcher Anspruch 5 oder 6, **dadurch gekennzeichnet, daß** die Mutation einer Substitution von Guanin G in der Position 2196 von SQ ID NO 1 durch eine Thymidin T entspricht, und zwar um einen zwischen den Positionen 2196 und 2198 von SEQ ID NO 1 liegenden Stopcodon TAA zu schaffen.

8. *E*. *coli*-Stammen nach irgendwelcher Anspruch 5 - 7, **dadurch gekennzeichnet, daß** das induzierbare Expressionssystem die Transkription einer DNA-Sequenz kontrolliert, die für einen oder mehreren bestimmten rekombinanten Polypeptide codiert.

9. Verfahren für die Herstellung von bestimmten rekombinanten Polypeptide, **dadurch gekennzeichnet, daß** es folgende Schritte beinhaltet :
- einen Veränderungsschritt von *E. coli*-Stammen, deren für die RNase E codierende Gen eine solche Mutation beinhaltet, daß das während der Expression von diesem mutierten Gen hergestellte Enzym nicht mehr die Abbauwirkung auf die mRNA besitzt, sondern die Reifungswirkung auf die ribosomale RNA (rRNA) der RNAse behaltet, worin diese Mutation das Wachstum der genannten *E. coli*-Stammen nicht beinflußt, **dadurch gekennzeichnet, daß** die Mutation aus der Substitution oder aus der Deletion von einem oder mehreren Nukleotiden der Genregion besteht, die für den C-terminalen Teil der RNase E zwischen dem Nukleotid in der Position 1935 und dem Nukleotid in der Position 3623 der DNA-Sequenz codiert, worin diese Sequenz selbst für die mit SEQ ID NO 1 dargestellte RNase codiert, und zwar um die Veränderung oder Deletion von zumindest einer Aminosäure bis zur Gesamtheit der 563 letzten Aminosäuren des C-terminalen Teils der mit SEQ ID NO2 dargestellen RNase E zu verursachen, mit einem Vektor, insbesondere einem Plasmid, der die für einen oder mehreren rekombinanten Polypeptide codierende Nukleotidsequenz beinhaltet,
- die Kultivierung der transformierten, während dem vorigen Schritt erhaltenen *E. coli-*Stammen, und zwar während einer ausreichenden Dauer, um die Expression des oder der rekombinanten Polypeptide in den *E*. *coli*-Zellen zu erlauben,
- und die Wiedererlangung des oder der während der vorigen Schritt hergestellten rekombinanten Polypeptide und zwar gegebenenfalls nach ihrer Reinigung, insbesondere durch Chromatographie, Elektrophorese oder selektive Ausfällung.

10. Verfahren für die Herstellung von bestimmten rekombinanten Polypeptiden nach Anspruch 9, **dadurch gekennzeichnet, daß** es die folgenden Schritte beinhaltet:
- einen Veränderungssehritt von *E*. *coli*-Stammen nach irgendwelcher Anspruch 1 - 7, mit einem Vektor, insbesondere einem Plasmid, der die für einen oder mehreren rekombinanten Polypeptide codierende Nukleotidsequenz beinhaltet, und zwar um *E. coli*-Stammen nach Anspruch 8 zu bekommen, worin die Transkription der für ein oder mehrere rekombinante Polypeptide genannten Nukleotidsequenz von einem induktierbaren Expressionssystem kontrolliert wird,
- die Kultivierung der während dem vorigen Schritt erhaltenen transformierten *E. coli-*Stammen, und die Induktion des genannten Expressionssystems, und zwar während einer ausreichender Dauer, um die Expression des oder der rekombinanten Polypeptide in den *E. coli*-Zellen zu erlauben,
- und die Wiedererlangung des oder der während der vorigen Schritt hergestellten rekombinanten Polypeptide.
